# EUROPEAN PATENT SPECIFICATION

(11) **EP 0 428 380 B1**
(45) Date of publication and mention of the grant of the patent: **13.10.1993**
(21) Application number: 90312376.8
(22) Date of filing: 13.11.1990
(51) Int. Cl.: A61M 15/00

(54) **Inhalation device and protective casing**
Inhalator und Schutzbehälter
Inhalateur et boîtier de protection

(30) Priority: 14.11.1989 GB 8925707
(43) Date of publication of application: 22.05.1991
(73) Proprietor: RIKER LABORATORIES, INC., Northridge, CA 91324 (US)
(72) Inventor: Wass, Anthony Charles Lammond, Stamford, Lincs., PE9 3QE (GB)
(74) Representative: Bowman, Paul Alan

(56) References cited:
- DE-A- 1 917 912
- FR-A- 2 069 300
- FR-A- 2 568 548

## Description

This invention relates to medical inhalers, and in particular to an improvement to the protective casing surrounding a metered dose inhaler, the casing comprising a body portion and a movable cover which, when displaced to allow the patient access to the device, acts as a cocking lever for the priming of the inhaler.

Medical inhalers comprising an aerosol vial containing propellant and medicament and equipped with a dispensing valve, e.g., a metered dose valve communicating with a mouthpiece, are known. Such inhalers may be incorporated in a housing including a breath actuated mechanism to synchronise dispensing of the medicament with inspiration by the patient. An example of such a device is commercially available from Minnesota Mining and Manufacturing Company, under the trade mark AUTOHALER and is disclosed, for example, in EP-A-147028.

FR-A- 2069300 discloses an inhalation device comprising an inhaler including a housing which comprises a mouthpiece and actuation means to prevent dispensing from the reservoir until a patient is ready to inhale through the mouthpiece and a protective casing surrounding the inhaler, the casing comprising a body portion and a movable cover pivotally attached to the casing which may be displaced to allow a patient access to the mouthpiece to use the inhaler, movement of the cover causing movement of a biasing means within the Protective casing thereby cocking the inhaler ready for use.

Co-pending European Patent Application 90309239.3 discloses an inhalation device comprising:
(i) a breath-actuated inhaler comprising a medicament reservoir mounted within a housing which comprises a mouthpiece and breath-actuation means which prevents dispensing from the reservoir until a patient inhales through the mouthpiece, and,
(ii) a protective casing surrounding the breath actuated inhaler, the casing comprising a body portion and a movable cover which may be displaced to allow a patient access to the mouthpiece to use the breath-actuated inhaler whilst it is within the casing, the breath-actuated inhaler being removable from the protective casing and operable outside the casing.

The present invention provides a cover arrangement which primes the inhaler for use upon opening the cover.

According to the present invention there is provided
(i) an inhaler including a housing which comprises a mouthpiece and actuation means to prevent dispensing from the reservoir until a patient is ready to inhale through the mouthpiece, and,
(ii) a protective casing surrounding the inhaler, the casing comprising a body portion and a movable cover which may be displaced to allow a patient access to the mouthpiece to use the inhaler, causing relative movement of the inhaler and a biasing means within the protective casing thereby cocking the inhaler ready for use, in which the cover is pivotally attached to said casing and a cocking link is pivotally mounted at one end to the cover wherein the cocking link has a portion in pivotal engagement with the inhaler or biasing means, whereby opening of the cover causes movement of the cocking link and inhaler relative to the biasing means, such that the pivot points of the cocking link and the pivot point of the cover to the casing pass through a straight line position to an overcentre position at which the inhaler device is cocked.

The cocking link provides a simple, robust and effective method of priming an inhaler for use, by coordinating the act of opening the casing cover with cocking of the inhaler mechanism.

Preferably the cocking mechanism includes guide means to define the movement of the portion of the cocking link engaging the inhaler or biasing means. Generally, the cocking link includes at least one guide arm, typically two, engaging a suitable slot or recess in the body portion of the casing. Thus, the direction and extent of movement of the cocking link portion (and therefore the inhaler or biasing means) is partly defined by both the direction and length of the recesses. In a preferred embodiment the cocking link comprises a wishbone bracket having two arms, each arm engaging a corresponding guide recess in the body portion of the protective casing. The cocking link preferably sacts directly on the inhaler.

The cover arrangement of the invention may be used with known metered dose or breath actuated pressurised inhalers. For a conventional pressurised inhaler comprising a cylindrical aerosol vial containing propellant and medicament and equipped with a dispensing valve, the inhaler is intended to be used in a substantially vertical position, in which the valve is lowermost relative to the vial. The cover may either be pivoted about a point lower than the inhaler, or about a point above the inhaler. Movement of the inhaler is generally completed in a substantially vertical direction, along the axis of the inhaler. The cover arrangement may also be used with dry powder devices which require priming prior to use by the patient.

The cover arrangement of the invention is found to possess a number of advantages, e.g.,:
(a) access to the aerosol dispenser and removal of the same, for cleaning purposes, freeing stem obstructions etc., is readily and simply effected without disassembly of the device;
(b) the cover when fully closed provides an effective seal restricting the ingress of contaminants, e.g., dirt or moisture;
(c) the cover is stable in the fully open position avoiding any tendency to close during use;
(d) when fully open, the cover is far removed from the proximity of the users facial extremities, and is resistant to breakage at the mounting point resulting from accident or clumsy handling, and,
(e) movement of the inhaler within the casing is completed in a straight line substantially free of obstructions and with reduced likelihood of jamming.

The invention will now be illustrated with reference to the accompanying drawings in which:
Figures 1 to 5 represent an inhalation device comprising a medical inhaler having a protective outer casing incorporating a cocking mechanism in accordance with the present invention;
Figures 1 and 4 illustrate sections through the device with the movable cover in the closed position and the inhaler uncocked;
Figures 2 and 3 illustrate sections through the device with the movable cover fully open and the inhaler cocked for use,
Figure 5 illustrates a front view of the device in the cocked position of Figures 2 and 3, and
Figures 6 and 7 represent partial sections through the protective casing of a device in accordance with the invention which is adapted to accommodate aerosol dispensers of different size.

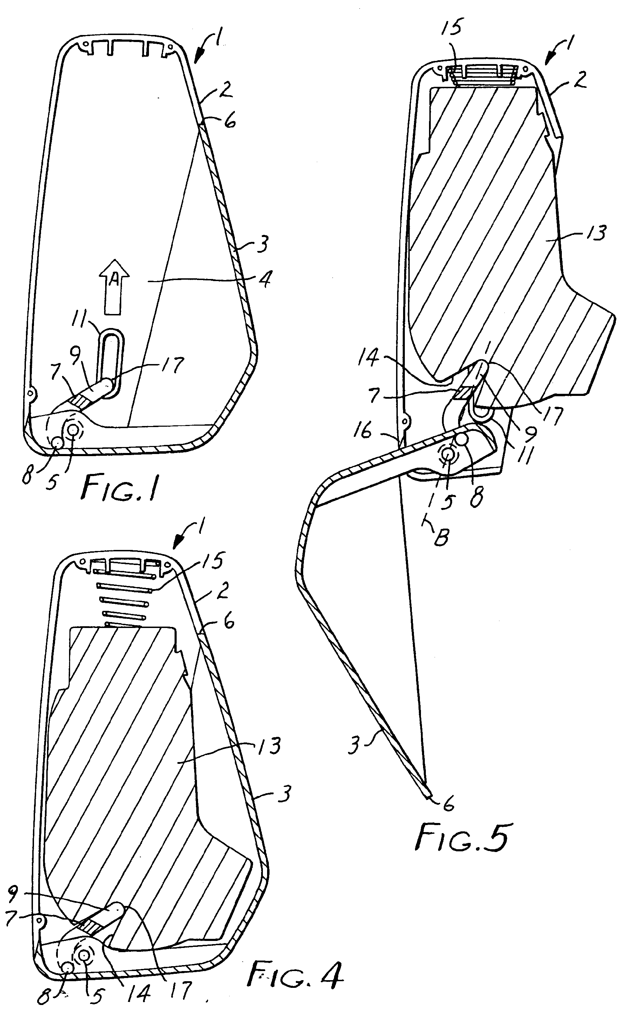

The aerosol dispenser is omitted in Figures 1 to 3 to more fully illustrate the cocking mechanism.

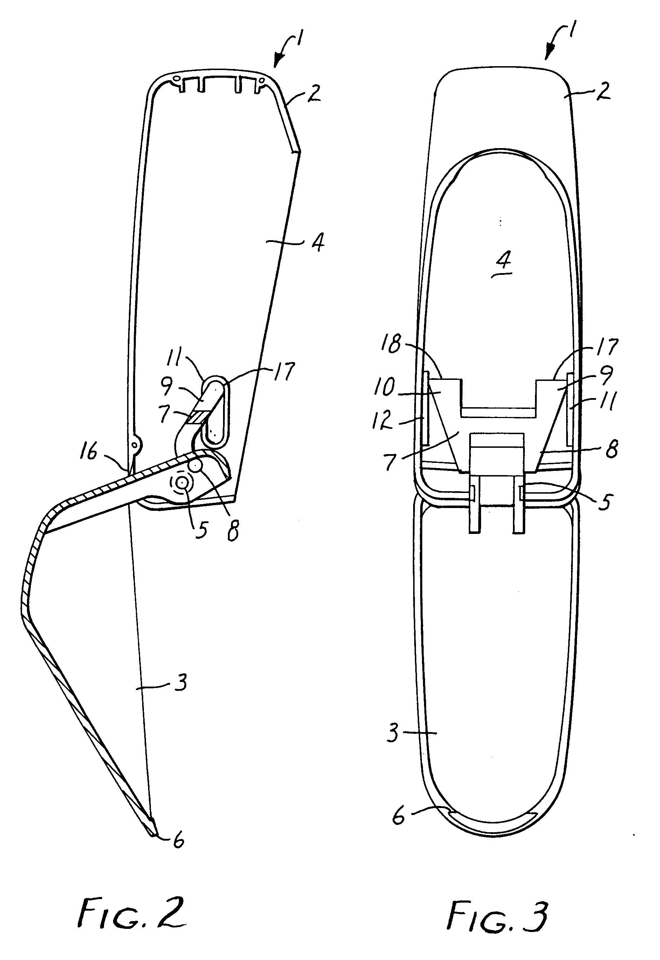

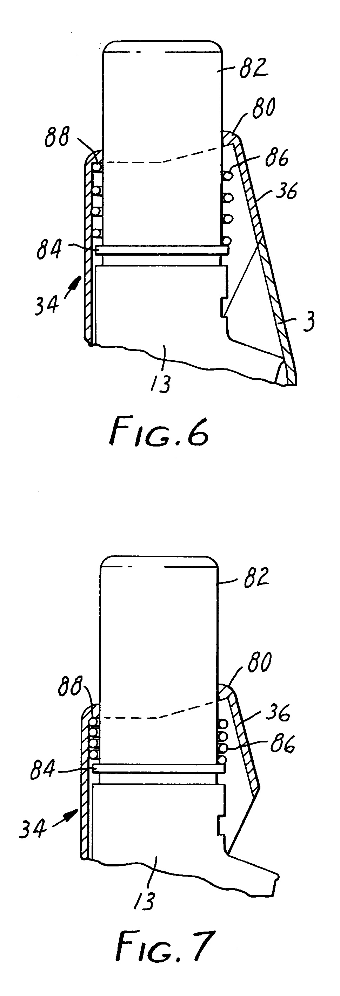

Referring to Figures 1 to 3 an inhalation device comprises a protective casing (1) adapted to receive a breath actuated aerosol dispenser, which casing comprises a body portion (2) and movable cover (3). Casing (1) defines a chamber (4), in which the aerosol dispenser (omitted for purposes of clarity) is located. Cover (3) is pivotally mounted about (5) allowing the patient to convert the device from an inactive closed format, in which the cover is in a home position (as depicted in Figures 1 and 4), to an open format in which the cover is fully displaced (as depicted in Figures 2, 3 and 5). The act of opening cover (3) provides the cocking force for the aerosol dispenser and allows the patient access to a suitable portal, such as a mouth or nasal adapter, through which medicament may be inhaled. The inhaler is maintained in the closed format while not in use providing a compact, convenient shape minimising contamination from dirt and moisture ingress etc. Cover (3) is advantageously provided with a snap fit (6) to positively retain the cover in its closed position.

The cocking mechanism comprises a wishbone bracket (7) which pivots about (8) on cover (3), such that opening of cover (3) drives bracket (7) from a home position (depicted in Figure 1) to a fully displaced position (depicted in Figures 2 and 3). The direction and extent of bracket displacement is defined by the engagement of bracket arms (9) and (10) with housing recesses (11) and (12) respectively. Recesses (11) and (12) are oriented such that displacement of cover (3) drives the bracket in a direction along the longitudinal axis of both casing and inhaler (represented by arrow 'A').

Referring to Figures 4 and 5, the aerosol dispenser (13) is located within chamber (4) by the provision of a groove (14) on the surface of dispenser (13) which pivotally engages the upper surfaces (17,18) of wishbone bracket arms (9) and (10) respectively, such that the aerosol container abuts against cocking spring (15), thereby stably seating the dispenser.

In use, the device is held in the hand such that the longitudinal axis of the body portion approximates to the vertical. Full displacement of cover (3) displaces bracket (7) to lift the dispenser in a straight vertical path, without any rubbing contact with the internal surface of the body portion, thereby compressing cocking spring (15). Subsequent relaxation of spring (15) upon device actuation, i.e. patient inspiration, provides the necessary force for displacing the aerosol vial relative to the outlet valve member. In an alternative embodiment, cocking spring (15) may be replaced by a deformable elastic member.

Body portion (1) and groove (14) are configured such that unwanted movement of the dispenser is prevented during device inversion. For example, body portion (1) may be provided with one or more longitudinal spacer ribs (not shown) which project from the inner body surfaces to restrict lateral movement of the dispenser during day to day transport or accidental dropping by the user.

The dispenser may be removed for cleaning, freeing stem obstructions or replacement of a new aerosol vial upon exhaustion of the old, by the user simply lifting the dispenser against spring (15), sufficient to disengage groove (14) from bracket arms (9) and (10) and withdrawing the dispenser through the cover opening.

The extent of bracket displacement and hence lift imparted to the dispenser is proportional to the extent of the initial opening of the cover. Maximum dispenser lift and therefore spring compression is completed by displacing the cover through about 150°, whereas fully opening the cover requires a displacement of about 165°. The user thus senses a stepped movement when displacing the cover. During the first 150° of displacement the cover works to compress the spring which reaches a maximum when pivot point (8) passes through a straight line position defined by the upper surface (17) of bracket arm (9) and pivot point (5) (illustrated by dotted line B; Figure 5), to an overcentre position at which the device is cocked.

The device may then be converted between two stable formats; (a) fully closed and (b) fully open. Any intermediary position for cover opening is inherently unstable, such that the casing will tend towards either of the stable formats, depending on which side of the 150° step the cover presently lies. This prevents the cover from inadvertently snapping shut on the user's facial extremities once fully opened and allows the cover to be far removed from obstructing the patient's chin. Additionally, the device is configured such that cover (3) abuts a stop (16) to provide greater resistance to breakage at the pivot of cover and housing as a result of mishandling or accidental dropping of the device.

The relative positions of the pivot points (5) and (10,9) allows the cover (3) to be shaped such that, when the cover is closed, the protective casing fully envelopes the inhaler restricting the ingress of contaminants.

Figures 6 and 7 of the accompanying drawings illustrate a breath-actuated inhaler in accordance with the invention in which the protective casing (34) may be modified to accommodate aerosol vials of different sizes. The body portion (36) of the casing has an aperture (80) through which a shroud (82) extends which accommodates the aerosol vial (not shown). A series of shrouds (82) may be fabricated having different lengths and, possibly, internal diameters, in order to accommodate various sizes of aerosol vial.

Whilst a cocking spring may be positioned within the top of the shroud (82) (in a similar manner to the cocking spring (15) shown in Figure 4), to absorb and retain the cocking force applied when the cover (3) is opened, a cocking spring external of the shroud (82) may be employed. The shroud (82) is provided with a flange (84) and cocking spring (86) is positioned around the shroud (82) extending between the flange (84) and a stop (88) at the top of the protective casing (36). When the cover (3) is opened, the breath-actuated inhaler, together with the shroud (82) is lifted (Figure 7) compressing cocking spring (86). When the patient breathes through the mouthpiece, the breath-actuated mechanism is triggered moving the shroud (82) and aerosol vial downwards to fire the aerosol valve.

In a further embodiment of the invention (not illustrated in the drawings) the shroud (82) shown in Figures 6 and 7 may be dispensed with and replaced by a circumferential flange extending around the aerosol vial, equivalent to flange (84), against which cocking spring (86) will act. The circumferential flange may be fabricated as a snap-on component around the aerosol vial e.g., in the region of the neck of the vial. This arrangement will obviate the need for fabricating a series of shrouds to accommodate the different sizes of aerosol vial, since the aerosol vial will simply extend through the top of the protective casing.

## Claims

1. An inhalation device comprising:
(i) an inhaler (13) including a housing which comprises a mouthpiece and actuation means to prevent dispensing from the reservoir until a patient is ready to inhale through the mouthpiece, and,
(ii) a protective casing (1) surrounding the inhaler, the casing comprising a body portion (2) and a movable cover (3) which may be displaced to allow a patient access to the mouthpiece to use the inhaler, causing relative movement of the inhaler (13) and a biasing means (15) within the protective casing (1) thereby cocking the inhaler ready for use, in which the cover (3) is pivotally attached (5) to said casing (1) and a cocking link (7) is pivotally mounted at one end (8) to the cover characterised in that the cocking link has a portion (17) in pivotal engagement with the inhaler or biasing means, whereby opening of the cover causes movement of the cocking link (7) and inhaler relative to the biasing means (15), such that the pivot points (8, 17) of the cocking link (7) and the pivot point (5) of the cover to the casing (5) pass through a straight line position to an overcentre position at which the inhaler device is cocked.

2. An inhalation device as claimed in Claim 1 in which the cocking link (7) is in pivotal engagement with the inhaler.

3. An inhalation device as claimed in Claim 1 or Claim 2 in which the inhaler comprises an aerosol vial containing propellant and medicament and equipped with a dispensing valve.

4. An inhalation device as claimed in Claim 1 or Claim 2 in which the inhaler comprises a dry powder inhaler.

5. An inhalation device as claimed in any preceding claim in which the device includes guide means (11, 12) to define the direction of movement of the portion (9, 10) of the cocking link engaging the inhaler.

6. An inhalation device as claimed in Claim 5 in which the cocking link includes at least one guide arm (9, 10) engaging a slot or recess (11, 12) in the protective casing to define the direction of movement of the portion of the cocking link engaging the inhaler.

7. An inhalation device as claimed in any preceding claim in which the cocking link (7) comprises a wishbone bracket having two arms (9, 10), each arm engaging a guide recess (11, 12) in the body portion of the protective casing.

8. An inhalation device as claimed in any preceding claim in which the movement of the inhaler is in the axial direction of the inhaler.

9. An inhalation device as claimed in any preceding claim in which the movable cover (3) pivots through at least 150° to the fully open position.

10. An inhalation device as claimed in any preceding claim in which the inhaler comprises a cylindrical vial and dispensing valve intended to be used in a substantially vertical position with the valve lowermost.

11. An inhalation device as claimed in any preceding claim in which the cover (3) is shaped such that when the cover is closed the protective casing (1) completely envelopes the inhaler (13) restricting the ingress of contaminants.

12. An inhalation device as claimed in any preceding claim in which the inhaler is breath actuated.

13. An inhalation device as claimed in any preceding claim in which the biasing means is selected from a compression spring (15) or a deformable elastic member.

14. An inhalation device as claimed in any preceding Claim in which the inhaler (13) comprises an aerosol vial and the protective casing comprises a shroud (82) surrounding the aerosol vial.

15. An inhalation device as claimed in Claim 14 in which the shroud (82) is movable within the remainder of the protective casing and is spring biased (86) to urge the aerosol vial towards a firing position.

## Patentansprüche

1. Inhalationsvorrichtung mit:
(i) einem Inhalator (13) mit einem Gehäuse, welches ein Mundstück und eine Betätigungseinrichtung aufweist, um ein Abgeben aus dem Reservoir zu verhindern, bis ein Patient bereit ist, durch das Mundstück zu inhalieren, und
(ii) einem den Inhalator umgebenden Schutzgehäuse (1) welches einen Hauptteil (2) und einen beweglichen Deckel (3) aufweist, welcher verschoben werden kann, um einem Patienten Zutritt zu dem Mundstück zur Benutzung des Inhalators zu erlauben, wobei eine Relativbewegung des Inhalators (13) und einer Vorspanneinrichtung (15) innerhalb des Schutzgehäuses (1) bewirkt wird, wodurch der Inhalator in gebrauchsfertigen Zustand gebracht wird, wobei der Deckel (3) mit dem Gehäuse (1) gelenkig (5) verbunden ist, und ein Spannhebel (7) an einem Ende (8) gelenkig mit dem Deckel verbunden ist,
dadurch gekennzeichnet, daß der Spannhebel einen Abschnitt (17) in gelenkigem Eingriff mit dem Inhalotor oder der Vorspanneinrichtung aufweist, wodurch ein Öffnen des Deckels eine Bewegung des Spannhebels (7) und des Inhalators relativ zu der Vorspanneinrichtung (15) bewirkt, so daß die Anlenkpunkte (8, 17) des Spannhebels (7) und der Anlenkpunkt (5) des Deckels an dem Gehäuse (1) durch eine geradlinige Stellung in eine Sprungstellung wandern, in der die Inhalationsvorrichtung gespannt steht.

2. Inhalationsvorrichtung gemäß Anspruch 1, wobei der Spannhebel (7) in gelenkigem Eingriff mit dem Inhalator steht.

3. Inhalationsvorrichtung gemäß Anspruch 1 oder 2, wobei der Inhalator ein Aerosolfläschchen aufweist, welches Treibmittel und Medikament enthält und mit einem Abgabeventil versehen ist.

4. Inhalationsvorrichtung gemäß Anspruch 1 oder 2, wobei der Inhalator einen Trockenpulverinhalator aufweist.

5. Inhalationsvorrichtung gemäß einem der vorhergehenden Ansprüche, wobei die Vorrichtung eine Führungseinrichtung (11, 12) aufweist, um die Bewegungsrichtung des mit dem Inhalator in Eingriff stehenden Teils (9, 10) des Spannhebels zu begrenzen.

6. Inhalationsvarrichtung gemäß Anspruch 5, wobei der Spannhebel mindestens einen Führungsarm (9, 10) aufweist, der mit einem Schlitz oder einer Ausnehmung (11, 12) in dem Schutzgehäuse in Eingriff steht, um die Bewe- gungsrichtung des mit dem Inhalator in Eingriff stehenden Teils des Spannhebels zu begrenzen.

7. Inhalationsvorrichtung gemäß einem der vorhergehenden Ansprüche, wobei der Spannhebel (7) einen Dreiecksträger mit zwei Armen (9, 10) aufweist, wobei jeder Arm mit einer Führungsausnehmung (11, 12) in dem Hauptteil des Schutzgehäuses in Eingriff steht.

8. Inhalationsvorrichtung gemäß einem der vorhergehenden Ansprüche, wobei die Bewegung des Inhalators in Axialrichtung des Inhalators stattfindet.

9. Inhalationsvorrichtung gemäß einem der vorhergehenden Ansprüche, wobei der bewegliche Deckel über mindestens 150° in die völlig geöffnete Stellung schwenkt.

10. Inhalationsvorrichtung gemäß einem der vorhergehenden Ansprüche, wobei der Inhalator ein zylindrisches Fläsch- chen und ein Abgabeventil aufweist, die zur Verwendung in einer im wesentlichen vertikalen Stellung mit dem Ventil zuunterst vorgesehen sind.

11. Inhalationsvorrichtung gemäß einem der vorhergehenden Ansprüche, wobei der Deckel (3) derart geformt ist, daß das Schutzgehäuse (1) den Inhalator (13) vollständig umgibt und das Eindringen von Verschmutzungen beschränkt, wenn der Deckel geschlossen ist.

12. Inhalationsvorrichtung gemäß einem der vorhergehenden Ansprüche, wobei der Inhalator durch den Atem betätigt wird.

13. Inhalationsvorrichtung gemäß einem der vorhergehenden Ansprüche, wobei die Vorspanneinrichtung eine Druckfeder (15) oder ein deformierbares elastisches Bauteil ist.

14. Inhalationsvorrichtung gemäß einem der vorhergehenden Ansprüche, wobei der Inhalator (13) ein Aerosolfläschchen aufweist und das Schutzgehäuse eine das Aerosolfläschchen umgebende Abschirmung (82) aufweist.

15. Inhalationsvorrichtung gemäß Anspruch 14, wobei die Abschirmung (82) mit dem Rest des Schutzgehäuses beweglich und federgespannt (86) ist, um das Aerosolfläschchen in eine Zündstellung zu drücken.

## Revendications

1. Dispositif d'inhalation comportant :
(i) un inhalateur (13) comprenant un corps qui comporte un embout et un moyen d'actionnement pour empêcher une distribution à partir du réservoir jusqu'à ce qu'un patient soit prêt à inhaler à travers l'embout, et
(ii) un boîtier protecteur (1) entourant l'inhalateur, le boîtier comportant une partie de corps (2) et un capot mobile (3) qui peut être déplacé pour permettre à un patient d'accéder à l'embout pour utiliser l'inhalateur, provoquant un mouvement relatif de l'inhalateur (13) et d'un moyen de rappel (15) à l'intérieur du boîtier protecteur (1), armant ainsi l'inhalateur prêt pour l'utilisation, le capot (3) étant relié de façon pivotante (5) audit boîtier (1) et une biellette (7) d'armement étant montée de façon pivotante par une extrémité (8) sur le capot, caractérisé en ce que la biellette d'armement comporte une partie (17) en engagement pivotant avec l'inhalateur ou le moyen de rappel, de manière qu'une ouverture du capot provoque un mouvement de la biellette (7) d'armement et de l'inhalateur par rapport au moyen (15) de rappel tel que les points de pivotement (8,17) de la biellette (7) d'armement et le point de pivotement (5) du capot sur le boîtier (5) passent par une position en ligne droite vers une position de basculement dans laquelle le dispositif inhalateur est armé.

2. Dispositif d'inhalation selon la revendication 1, dans lequel la biellette (7) d'armement est en engagement pivotant avec l'inhalateur.

3. Dispositif d'inhalation selon la revendication 1 ou la revendication 2, dans lequel l'inhalateur comprend un flacon à aérosol contenant un propulseur et un médicament et pourvu d'une valve de distribution.

4. Dispositif d'inhalation selon la revendication 1 ou la revendication 2, dans lequel l'inhalateur comprend un inhalateur à poudre sèche.

5. Dispositif d'inhalation selon l'une quelconque des revendications précédentes, dans lequel le dispositif comprend des moyens de guidage (11,12) destinés à définir la direction du mouvement de la partie (9,10) de la biellette d'armement engageant l'inhalateur.

6. Dispositif d'inhalation selon la revendication 5, dans lequel la biellette d'armement comprend au moins un bras de guidage (9,10) engageant une rainure ou un évidement (11,12) dans le capot protecteur pour définir la direction du mouvement de la partie de la biellette d'armement engageant l'inhalateur.

7. Dispositif d'inhalation selon l'une quelconque des revendications précédentes, dans lequel la biellette (7) d'armement comporte un support en fourchette ayant deux bras (9,10), chaque bras engageant un évidement (11,12) de guidage situé dans la partie de corps du boîtier protecteur.

8. Dispositif d'inhalation selon l'une quelconque des revendications précédentes, dans lequel le mouvement de l'inhalateur est dans la direction axiale de l'inhalateur.

9. Dispositif d'inhalation selon l'une quelconque des revendications précédentes, dans lequel le capot mobile (3) pivote sur au moins 150° vers la position d'ouverture complète.

10. Dispositif d'inhalation selon l'une quelconque des revendications précédentes, dans lequel l'inhalateur comprend un flacon cylindrique et une valve de distribution prévus pour être utilisés dans une position sensiblement verticale, la valve étant au point le plus bas.

11. Dispositif d'inhalation selon l'une quelconque des revendications précédentes, dans lequel le capot (3) est configuré de manière que, lorsque le capot est fermé, le boîtier protecteur (1) enveloppe complètement l'inhalateur (13), faisant obstacle à l'entrée de contaminants.

12. Dispositif d'inhalation selon l'une quelconque des revendications précédentes, dans lequel l'inhalateur est actionné par la respiration.

13. Dispositif d'inhalation selon l'une quelconque des revendications précédentes, dans lequel le moyen de rappel est choisi entre un ressort (15) de compression et un élément élastique déformable.

14. Dispositif d'inhalation selon l'une quelconque des revendications précédentes, dans lequel l'inhalateur (13) comporte un flacon d'aérosol et le boîtier protecteur comporte une jupe (82) entourant le flacon d'aérosol.

15. Dispositif d'inhalation selon la revendication 14,dans lequel la jupe (82) est mobile à l'intérieur de la partie restante du boîtier protecteur et est rappelée par ressort (86) pour solliciter le flacon d'aérosol vers une position de déclenchement.
